Europäisches Patentamt

European Patent Office

Office européen des brevets

(11) Veröffentlichungsnummer: **0 137 115**

A1

# (12) EUROPÄISCHE PATENTANMELDUNG

(21) Anmeldenummer: 84107163.2

(22) Anmeldetag: 22.06.84

(51) Int. Cl.⁴: $A\ 61\ L\ 27/00$
$A\ 61\ F\ 2/30$

(30) Priorität: 06.07.83 DE 3324256

(43) Veröffentlichungstag der Anmeldung:
17.04.85 Patentblatt 85/16

(84) Benannte Vertragsstaaten:
CH DE FR GB LI

(71) Anmelder: ROBERT BOSCH GMBH
Postfach 50
D-7000 Stuttgart 1(DE)

(72) Erfinder: Baur, Peter, Dr. Dipl.-Ing.
Gablenberger Hauptstrasse 146c
D-7000 Stuttgart 1(DE)

(72) Erfinder: Gohl, Walter, Dr. Dipl.-Phys.
Kniebisstrasse 26
D-7031 Aidlingen(DE)

(72) Erfinder: Kohlberger, Gerold
Galgenberg 3
D-7050 Waiblingen(DE)

(72) Erfinder: Petsch, Franz
Römerstrasse 134
D-7250 Leonberg(DE)

(54) Endoprothese aus faserverstärktem duroplastischem Kunstharz und Verfahren zur Herstellung.

(57) Es werden eine Endoprothese aus faserverstärktem duroplastischem Kunstharz sowie Verfahren zur Herstellung derselben vorgeschlagen, die auf den vor allem Biegekräften ausgesetzen Seiten Gurte aus faserverstärktem duroplastischem Kunstharz mit unidirektionaler Faserrichtung aufweisen. Zwischen diesen Gurten befindet sich ein faserverstärkter duroplastischer Kunststoff mit einem gerichteten Faservlies, einem Wirrvlies oder einer faserhaltigen Preßmasse. Bei den Verfahren zur Herstellung dieser Endoprothese ist es vor allem wichtig, daß die aus dem faserverstärkten duroplastischen Kunststoff mit unidirektionaler Faserrichtung bestehenden Gurte bei der weiteren Verarbeitung ihre ausgerichtete Lage beibehalten. Dies geschieht vor allem dadurch, daß man dafür sorgt, daß der Vorvernetzungsgrad der Gurte wesentlich höher ist derjenige des zwischen den Gurten eingebrachten Materials. Die Herstellung der Endoprothesen erfolgt vorzugsweise über die Herstellung eines Vorformlings, der noch mit weiteren faserhaltigen Schichten umgeben werden kann, kann aber auch in einer Preßform in einem einzigen Arbeitsgang erfolgen.

R. 18782

1.7.1983

0137115

ROBERT BOSCH GMBH, 7000 Stuttgart 1

Endoprothese aus faserverstärktem duroplastischem
Kunstharz und Verfahren zur Herstellung

Stand der Technik

Die Erfindung geht aus von einer Endoprothese nach der
Gattung des Anspruchs 1 sowie von Verfahren nach der
Gattung der Verfahrensansprüche. Aus der DE-OS 29 41 369
sind Endoprothesen aus faserverstärktem duroplastischem
Kunstharz bekannt, bei denen zum Aufbau entweder ausschließlich Vliese aus Kohlenstoffasern mit unterschiedlicher Orientierung der Fasern oder aber abwechselnd
Lagen aus Vliesen und Langfasern eingesetzt werden, wobei sich die einzelnen Lagen aus Vlies- bzw. Langfasern
durch das ganze Formteil hindurch abwechseln. Es hat
sich hier jedoch gezeigt, daß die Festigkeitseigenschaften dieser in erster Näherung homogen aufgebauten Formteile den Anforderungen noch nicht genügten.

Vorteile der Erfindung

Die erfindungsgemäße Endoprothese mit den kennzeichnenden
Merkmalen des Anspruchs 1 hat demgegenüber den Vorteil,
daß sie dort, wo die größten Biegekräfte auftreten, aus
unidirektionalen Gurten besteht, die auf der Ober- und
Unterseite des Formteiles durchlaufen und Biegekräfte

...

besonders gut aufzunehmen in der Lage sind, während der
Raum zwischen den Gurten mit kurzfaserigem Verbundwerkstoff ausgefüllt ist, der hauptsächlich Druck- und
Schubbeanspruchungen aufnimmt. Dabei kann dieser zwischen den Gurten eingebrachte Verbundwerkstoff je nach
Belastungshöhe gerichtetes Faservlies oder Wirrvlies
enthalten oder aus Preßmasse mit Kurzfasern bestehen.
Besonders günstig ist es, wenn das duroplastische
Kunstharz ein Triazinharz ist und die Fasern aus Kohlenstoff bestehen. Aus der obengenannten DE-OS 29 41 369
ist nämlich auch bekannt, daß das Triazinharz eine hervorragende Körperverträglichkeit aufweist und diese
Körperverträglichkeit sich in Kombination mit Kohlefasern nicht ändert.

Durch die in den Verfahrensansprüchen angegebenen Merkmale lassen sich die erfindungsgemäßen Endoprothesen in
wirtschaftlicher Weise und unter reproduzierbaren Fertigungsbedingungen herstellen, so daß eine gleichbleibende Qualität der nach den Verfahrensansprüchen hergestellten Endprothesen gewährleistet ist.

Beschreibung eines Ausführungsbeispiels

Zur Herstellung eines Femurschaftes einer Hüftgelenk-
Endoprothese werden zunächst zur Bildung der Gurte
Kohlefaserstränge oder Rovings mit einem Triazin-Flüssigharz, z. B. Triazin A der Fa. Bayer AG, welches dem
BT-Resin 2060 der Fa. Mitsubishi Gas Chemical Comp.,
Inc., entspricht, gelöst in Azeton (70 Gew.-% Harz in
30 Gew.-% Azeton) getränkt und in einer Dicke von etwa
5 - 10 mm in eine auf ca. 120 $^{\circ}$ C aufgeheizte Form, die
dem herzustellenden Femurschaft in etwa entspricht,

...

- 3 -

eingelegt, derart, daß die Ober- und Unterseite des
Femurschaftes von diesen Rovings gebildet wird und
die Faserrichtung der Längachse des Femurschaftes entspricht. In den verbleibenden Zwischenraum zwischen
diesen imprägnierten Rovings werden sodann Richtfaser-
oder Wirrvliesstücke, die ebenfalls mit dem flüssigen
Triazinharz getränkt sind, eingelegt. Das Einlegen
der Rovings in die vorgeheizte Form entlang der Wandungen hat zur Folge, daß diese bereits vorvernetzt
werden, so daß der Vorvernetzungsgrad der Rovings
größer ist als derjenige der Vliese. Dadurch wird erreicht, daß beim nun folgenden Schließen der Form die
Rovings ihre ausgerichtete Lage beibehalten und durch
das leichter fließende Vlies verdichtet werden. Im Anschluß daran läßt man die Form auf etwa 60 $^{\circ}$C abkühlen
und entnimmt nun den so gebildeten Vorformling. Zum
Aushärten des Vorformlings legt man diesen in ein Preßwerkzeug ein, das die Form der Endoprothese aufweist.
Vor diesem Einlegen in das Preßwerkzeug können zur Erhöhung der Schub- und Torsionssteifigkeit noch zusätzliche Gewebelagen aufgebracht oder mit Harz imprägnierte
Schläuche aus Kohlenstoffasern über den Vorformling gestreift werden. In diesen Fällen ist der Vorformling
dann entsprechend kleiner als die endgültige Endoprothese. Das Preßwerkzeug wird nach dem Einlegen des
gegebenenfalls so ergänzten Vorformlings geschlossen
und auf eine Temperatur von 160 $^{\circ}$C gebracht, so daß der
Vorformling aushärtet. Nach dem Aushärten der Form kann
das fertige Formteil dann entnommen werden.

Obwohl die soeben beschriebene Art der Herstellung die
bevorzugte ist, ist es, vor allem wenn an die Festigkeitseigenschaften keine zu hohen Anforderungen gestellt
werden, auch möglich, statt des Einlegens von Richtfaser-

...

oder Wirrvliesstücken in den Zwischenraum zwischen den beiden Gurten nach dem Einlegen der imprägnierten Kohlefaser-Rovings die Form zu schließen und dann eine Formmasse aus Kunstharz und Wirrvlies oder Richtfaservlies oder Kurzfasermasse einzuspritzen. Die weiteren Schritte verlaufen in der gleichen Weise, wie sie oben beschrieben sind.

Schließlich ist es auch möglich, in das Preßwerkzeug nacheinander die äußeren Gewebelagen, dann die mit Kunstharz getränkten Faserstränge und schließlich entweder in die offene Form die mit Kunstharz getränkten Richtfaser- oder Wirrvliesstücke in die Zwischenräume einzulegen oder in die geschlossene Form eine Formmasse aus Kunstharz und Wirrvlies oder Richtfaservlies oder Kurzfasern einzuspritzen und die geschlossene Form dann auf eine Temperatur von 160 $^{\circ}$C aufzuheizen, um das so gebildete Formteil auszuhärten. Auch hier wird vor dem Einlegen bzw. Einspritzen das Preßwerkzeug auf eine Temperatur von 120 $^{\circ}$C aufgeheizt.

R. **18782**

1.7.1983 Pf/Jä

0137115

ROBERT BOSCH GMBH, 7000 Stuttgart 1

Ansprüche

1. Endoprothese aus faserverstärktem duroplastischem
Kunstharz, dadurch gekennzeichnet, daß sie auf den
vor allem Biegekräften ausgesetzten Seiten Gurte aus
faserverstärktem duroplastischem Kunststoff mit unidirektionaler Faserrichtung und dazwischen einen faserverstärkten duroplastischen Kunststoff mit einem
gerichteten Faservlies, einem Wirrvlies oder einer
faserhaltigen Preßmasse aufweist.

2. Endoprothese nach Anspruch 1, dadurch gekennzeichnet,
daß das duroplastische Kunstharz ein Triazinharz ist
und die Fasern aus Kohlenstoff bestehen.

3. Verfahren zur Herstellung von Endoprothesen nach Anspruch 1 oder 2, gekennzeichnet durch folgende Verfahrensschritte:

    a. Einlegen von mit Kunstharz imprägnierten unidirektionalen Fasersträngen (Rovings) in eine
       auf eine Temperatur niedriger als die Aushärtetemperatur aufgeheizte Form entlang der Ober-
       und Unterseite der Formhöhlung zur Bildung der
       Gurte,

    b. Einlegen von mit Kunstharz getränkten Richtfaser- oder Wirrvliesstücken in den so gebil-
       deten Zwischenraum, wobei der Vorvernetzungs-

. . .

grad der in Schritt a. eingelegten imprägnierten
Faserstränge größer ist als derjenige des gemäß
b. eingebrachten Materials;

c. Schließen der Form,

d. Entnahme des so gebildeten Vorformlings aus der
   auf ca. 60 $^{\circ}$C abgekühlten Form,

e. Einlegen des Vorformlings in ein Preßwerkzeug,
   nachdem zuvor gegebenenfalls zusätzliche Gewebelagen oder mit Kunstharz imprägnierte Faserschläuche um den Vorformling gelegt wurden,
   und Aushärten des Vorformlings in dem Preßwerkzeug bei der Aushärttemperatur unter Bildung
   des Formteiles.

4. Verfahren nach Anspruch 3, dadurch gekennzeichnet,
daß anstelle der Verfahrensschritte b., c. in die nach
dem Verfahrensschritt a. geschlossene Form eine Formmasse
aus Kunstharz und Wirrvlies oder Richtfaservlies oder
Kurzfasern eingespritzt werden, wobei der Vorvernetzungsgrad der eingelegten imprägnierten Faserstränge größer
ist als derjenige der Formmasse, wonach die Verfahrensschritte d. und e. folgen.

5. Verfahren nach Anspruch 3 oder 4, dadurch gekennzeichnet, daß anstelle der Verfahrensschritte d. und e.
zunächst die Gewebelagen der Verfahrensstufe e., dann
die mit Kunstharz getränkten Faserstränge der Verfahrensstufe a. in eine Preßform eingelegt werden, wonach entweder in die offene Form mit Kunstharz getränkte Richtfaser- oder Wirrvliesstücke in die Zwischenräume eingelegt werden oder in die geschlossene Form eine Formmasse
aus Kunstharz und Wirrvlies oder Richtfaservlies oder
Kurzfasern eingespritzt wird, woraufhin die verschlossene Form auf die Aufheiztemperatur gebracht wird, um
das in der Preßform gebildete Formteil auszuhärten.

...

6. Verfahren nach einem der Ansprüche 3 bis 5, dadurch gekennzeichnet, daß das Kunstharz ein Triazinharz ist und die Fasern aus Kohlenstoff bestehen, und daß die Aushärtung bei einer Temperatur von ca. 160 $^{\circ}$C vorgenommen wird.

# EUROPÄISCHER RECHERCHENBERICHT

Europäisches
Patentamt

## EINSCHLÄGIGE DOKUMENTE

EP 84107163.2

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| D,A | <u>DE - A1 - 2 941 369</u> (R. BOSCH GMBH)<br><br>* Patentansprüche 1,3,4,5,6; Seite 6 *<br><br>-- | 1-3,5 | A 61 L 27/00<br>A 61 F 2/30 |
| A | <u>DE - A1 - 2 603 456</u> (R. BOSCH GMBH)<br><br>* Patentansprüche 1,2,6,8,12 *<br><br>-- | 1-3,5 | |
| A | <u>DE - A1 - 2 920 336</u> (SIGRI ELEKTR.)<br>* Gesamt *<br><br>-- | 1,3,5 | |
| A | <u>AT - B - 349 620</u> (SIGRI ELEKTR.)<br>* Gesamt *<br><br>-- | 1,3,5 | |
| A | <u>DE - B2 - 2 138 146</u> (R.F. HOCHMAN)<br>* Gesamt *<br><br>---- | 1 | RECHERCHIERTE SACHGEBIETE (Int. Cl.4)<br><br>A 61 F 1/00 |

Der vorliegende Recherchenbericht wurde für alle Patentansprüche erstellt.

| Recherchenort | Abschlußdatum der Recherche | Prüfer |
|---|---|---|
| WIEN | 26-11-1984 | LUDWIG |